# EUROPEAN PATENT APPLICATION

(11) **EP 1 938 807 A1**
(43) Date of publication of application: **02.07.2008**
(21) Application number: 06026646.7
(22) Date of filing: 22.12.2006
(51) Int. Cl.: A61K 9/50, A61K 9/16

(54) **Reactive powdering**

(71) Applicant: BASF SE, 67056 Ludwigshafen (DE)
(72) Inventor: End, Lutz, 68526 Ladenburg (DE); Hansen, Morten, Mohr, 3450 Alleroed (DK); Yde, Birgitte, 3520 Farum (DK)
(74) Representative: Benthin, Stig

(57) **Abstract**

The invention relates to microcapsules comprising one or more active substances embedded in a matrix material and comprising a powdering agent, said microcapsule further comprising a component which is the product of the reaction between at least one component in said matrix material and at least one component in said powdering agent, and which microcapsule is obtainable by a process which comprises the steps of
- providing a solution or dispersion of said matrix material,
- adding to said solution or dispersion said at least one active substance,
- treating the mixture thus obtained to prepare a solution or dispersion of said at least one active substance and said matrix material,
- finely dividing and drying the solution or dispersion thus obtained to prepare a mass of particles each containing the active substance embedded in the matrix material,
- adding simultaneously with said finely dividing and drying step said powdering agent,
whereby said powdering agent comprises said at least one component, which is able to react with said at least one component in said matrix component, as well as the method itself.

The microcapsules of the invention may be used in products, such as foods, food supplements, beverages, pharmaceutical or veterinary products, feeds or feed supplements, personal care products or household products.

## Description

### FIELD OF INVENTION

The present invention relates to microcapsules comprising one or more active substances embedded in a matrix material, a process of preparing such microcapsules as well as its use.

### BACKGROUND OF THE INVENTION

Naturally occurring and modified polysaccharides and naturally occurring hydrocolloids such as alginate, caseinate, carrageenan, gelatine, pectins, gum acacia, starch and modified starch, find wide spread use as matrix materials for use in the microencapsulating of sensitive or labile active substances, such as vitamins, aroma and flavour substances in food, food supplements, pharmaceuticals and agricultural products, in order to protect them from exposure to oxygen, moisture and irradiation as well as physical influences, such as pressure, and thus to avoid chemical and/or physical degradation of said active substances and to improve their storage stability.

However, there is a constant need of providing novel microcapsules, also by including alternative matrix materials, which have the attractive properties necessary for further processing into food, food supplements, pharmaceuticals and agricultural products.

One method for encapsulating water-insoluble substances in a starch derived matrix material is described in US Patent No. 3 455 838. This method comprises the making of an aqueous dispersion of the encapsulating agent, emulsifying the water-insoluble substance in said dispersion and drying the resulting emulsion to form dry, free-flowing particles. The drying of the emulsion is performed in e.g. a commercial spray-dryer.

A similar process is described in WO 91/06292, which discloses a process of preparing a hydrophobic/aerophilic solid, which can be dispersed in the water in the form of discrete microparticles. This method comprises the steps of milling the solid in an aqueous medium in the presence of a hydrocolloid to obtain a suspension containing suspended particles and finely dividing and drying of the suspension to provide a free-flowing powder. The finely dividing and drying is performed by conventional methods, such as spray cooling and spray drying. A powdering agent is preferably blown into the spraying chamber in order to prevent agglomeration of the formed microcapsules and to prevent adherence to the chamber wall.

Yet a similar method is described in EP Patent Application 922 449.

During these well known processes for preparing microcapsules containing an active substance embedded in a matrix material it may be opportune and suitable to include a component in the matrix material which can interact with a component in the powdering agent in order to obtain a reaction product giving the final product, viz. the microcapsules, desirable or required attractive properties, which can normally not be obtained by the traditional, well-known method.

The object of the present invention is to provide a novel process of preparing microcapsules with improved properties comprising one or more active substances embedded in the matrix material.

It is a further object of the invention to provide improved microcapsules having improved properties, such as hardness, improved mechanical structure or strength, improved resistance in a humid environment and/or improved stability against oxidation. Such improved properties lead to an improved performance during further processing of the microcapsules into food, food supplements, pharmaceuticals and agricultural products.

### SUMMARY OF THE INVENTION

The present invention relates accordingly to microcapsules comprising one or more active substances embedded in a matrix material and comprising a powdering agent, said microcapsules further comprising a component which is the product of the reaction between at least one component in said matrix material and at least one component in said powdering agent, and which microcapsule is obtainable by a process which comprises the steps of
- providing a solution or dispersion of said matrix material,
- adding to said solution or dispersion said at least one active substance,
- treating the mixture thus obtained to prepare a solution or dispersion of said at least one active substance and said matrix material,
- finely dividing and drying the solution or dispersion thus obtained to prepare a mass of particles each containing the active substance embedded in said matrix material,
- adding simultaneously with said finely dividing and drying step said powdering agent,
and further characterised in that said powdering agent comprises said at least one component, which is able to react with at least one component in said matrix component.

The invention further relates to a process of preparing such microcapsules, which process comprises the steps of
- providing a solution or dispersion of said matrix material,
- adding to said solution or dispersion said at least one active substance,
- treating the mixture thus obtained to prepare a solution or dispersion of said at least one active substance and said matrix material,
- finely dividing and drying the solution or dispersion thus obtained to prepare a mass of particles each containing the active substance embedded in said matrix,
- adding simultaneously with said finely dividing and drying step said powdering agent,
which process is characterised in that said powdering agent comprises said at least one component, which is able to react with said at least one component in said matrix component.

It has surprisingly been found that by including one or more suitable components in the matrix material and one or more suitable components in the powdering agent it is possible to achieve a reaction between the components. Such reaction is impossible or at least very difficult to achieve in the traditional process for preparing microcapsules, wherein the solution or dispersion of the matrix material typically comprises all relevant components.

The reaction product will typically form a layer, such as a film, complex or coating, on the surface of the microcapsules, which provide improved properties to the microcapsules, such as hardness, improved mechanical structure or strength, improved resistance in a humid environment and/or improved stability against oxidation.

The solution or dispersion of the matrix material and active substance are finely divided and simultaneously the powdering agent comprising another reaction component is added thus leading to a reaction. Subsequently, the material is dried. Since the finely dividing, the adding of powdering agent and the drying take place in a single process step, the process becomes an all-in-one process, which is simple and non-expensive.

The term "microcapsules" as used herein means particles each comprising a matrix material having embedded therein a plurality of solid or liquid micro particles or solute molecules. Microcapsules usually have a mean diameter of about 5 mm or smaller, e.g. between 1 mm and 0.05 mm, such as between 0.6 and 0.1 mm. They can also have a diameter e.g. between 2 mm and 0.01 mm, such as between 1.5 mm and 0.2 mm.

The term "dispersion" as used herein covers both an emulsion meaning a mixture comprising liquid particles (e.g. oil droplets) dispersed in a liquid medium, e.g. water, or a suspension meaning solid particles dispersed in a liquid medium, e.g. water.

The term "reaction" as used herein covers any chemical, physical or other reaction between suitable matching components.

### DETAILED DESCRIPTION OF THE INVENTION

The at least one matrix component and the at least one component in the powdering agent, which is able to react to provide the further component in the microcapsules, may for example be selected from proteins, enzymes, anions, cations, acids, bases, aldehydes, cross-linking agents, such as dialdehydes and enzymes; polycations, polyanions, electrolytes and water.

It is not important which of the components meant to participate in the reaction aimed at are present in the matrix, and which are present in the powdering agent. What is important is that the components are selected in a suitable way so that the intended reaction can take place during the finely dividing and drying step.

In a first embodiment of the invention one component is a protein, and another component is a reducing sugar. The protein and the reducing sugar enter a Maillard reaction, which provides an anti oxidizing effect in the resulting microcapsules and a cross-linking/hardening.

In a second embodiment one component is a protein, and another component is a dialdehyde. The dialdehyde induces a cross binding of the protein, thereby providing an improved mechanical structure in the resulting microcapsules.

In a third embodiment one component is a protein, and another component is an enzyme, such as transglutaminase. In this case the enzyme induces a cross binding of the protein, thereby providing an improved mechanical structure in the resulting microcapsules.

In a forth embodiment one component is a polysaccharide, such as alginate, and another component is a cation, e.g. a divalent cation such as calcium or another alkali metal or alkaline-earth metal ion. In this case the polysaccharide and the cations participate in a complex formation, thereby providing increased particle hardness to the resulting microcapsules.

In a fifth embodiment one component is a protein, and another component is water. In this case the protein is moistened with water, induced by heat and thereby either denaturated or dissolved, which leads to the formation of a protecting film layer on the surface of the microcapsules.

In a sixth embodiment one component is a protein, such as casein, and another component is an acid. The acid causes the casein to flocculate and denaturate to form a film layer on the surface of the microcapsules then acting as an oxygen/humidity barrier.

Depending on the participating components provided from respectively the matrix material and the powdering agent, the process conditions can be selected so as to promote the intended reaction between the components.

The step of treating the mixture to prepare a solution or dispersion of particles of at least one active substance and at least one matrix component in said aqueous medium may be carried out by any convenient method, such as mixing, homogenising, emulsifying, milling or dispersing.

The process step of finely dividing and drying the solution or dispersion to prepare a mass of particles each containing the active substance embedded in the matrix material may be carried out with conventional methods allowing the simultaneous addition of a powdering agent, such as spray cooling, spray drying, modified spray drying or sheet drying and crushing, see for example WO 91/06292.

The matrix material of the invention may be any conventional matrix material, such as naturally occurring and modified polysaccharides and naturally occurring hydrocolloids, e.g. alginate, carrageenan, gelatine, pectins and gum acacia, including the matrix component(s), which are intended to react with the powdering agent component. Said matrix component(s) may be the only matrix material present, or it/they may be component(s) present in addition to other matrix materials.

Starch derived from a natural source, such as potato, wheat, maize, tapioca and rice, and modified starch are other examples of suitable matrix materials. Modified starch prepared from the unmodified starch by (partial) degradation, depolymerisation, hydrolysis, etc is one appropriate matrix material. A very appropriate modified starch to be used according to the invention is OSA modified starch, e.g. sodium octenyl succinate modified starch, i.e. a starch wherein a part of the hydroxyl groups are esterified by octenyl succinyl acid, providing a surface active property of the starch.

The solution of the matrix material can optionally further contain excipients, such as hydrocolloids, dissolved carbohydrates, e.g. sorbitol and sucrose, and/or an antioxidant or oil being or containing an antioxidant.

The powdering agent to be used according to the invention may be any convention powdering agent, such as a starch, e.g. corn starch, a modified starch, tri-calcium phosphate, lactose, mannitol, ethylcellulose, coagulated albumin, hardened gelatine, casein, stearate-Ca, stearate-Na, a metal soap, hydrogenated ricinus oil, polyoxide, talcum, a wax, silica or a silicate.

The active substances comprised in the microcapsules of the invention or microcapsules prepared according to the invention may be any substance, e.g. any substance which during storage, transport, handling and use requires protection, e.g. from oxygen, moisture, light radiation, and physical influences, in order to avoid physical and chemical decomposition of the substance. These active substances are further defined as being active in either a chemical or biological system. A protective matrix may be used to prevent the active substance from reacting with other substances present in a composition or with substances with which it may come into contact during use and which would have a deleterious effect upon the active substance's desired property. Also, a protective matrix may be used to transform liquids and other substances, which are difficult to handle, e.g. due to stickiness, into a solid form suitable for handling and processing during use, such as a powder of microcapsules.

Examples of active substances suitable for use in connection with the present invention are:
Fat-soluble substances, such as vitamins, e.g. vitamin A and esters thereof, E and esters thereof, e.g. E-acetate, D and K, fatty acids (both of natural origin and obtained through a fermentative process), e.g. mono- and polyunsaturated fatty acids, which may be added in the form of fish oil containing i.a. the (n-3) fatty acids docosahexaenoic acid (DHA) and eicosapentaenoic acid (EPA), conjugated linolenic acid (CLA), phospholipids derived from e.g. egg yolk and in the form of evening primrose oil and castor oil containing i.a. the (n-6) fatty acid γ-linolenic acid, arachidonic acid, lipoic acid, carotenoids, e.g. β-carotene, lutein, lycopene, β-cryptoxanthin, astaxanthin, Cantaxanthin, citranaxanthin and zeaxanthin, curcumin, benzoquinones, e.g. Coenzyme Q10 (ubidecarenone), phytosterols, oils and fats;
Water-soluble substances, such as vitamin B and C;
Enzymes, e.g. amylase;
Pharmaceuticals, such as griseofulvin, ibuprofen, benzodiazepines, phenacetin, hormones and paracetamol; and
Nutritional supplements, such as minerals and isoflavones.

The matrix material may further contain conventional additives such as antioxidants, e.g. t-butylhydroxytoluene (BHT), t-butylhydroxyanisole (BHA), ascorbic acid, ascorbyl palmitate, sodium ascorbate, citric acid, sodium citrate, EDTA or its safts, tocopherols, TBHQ, ethoxyquine, propyl gallate, and extracts from herbs, i.a. rosemary or oregano extract; anti-caking agents, e.g. tri-calcium phosphate and silicates, i.a. silicon dioxide and sodium aluminium silicate; plasticizers, e.g. carbohydrates and carbohydrate alcohols, examples of which are saccharose, glucose, fructose, lactose, invert sugar, sorbitol, mannitol, Trehalose, Tagatose, Pullulan, Raftilose (oligofructose), dextrin, maltodextrin, glycerin, and mixtures thereof, such as saccharose, Trehalose, Pullulan, dextrin and Raftilose and mixtures thereof.

The matrix material may further contain conventional additives such as emulsifiers and surfactants, e.g. ascorbyl palmitate, mono- and diglycerides of fatty acids and derivatives thereof, and lecithin.

The microcapsules according to the invention are suitable for a wide variety of applications, such as food, food supplements, beverages, pharmaceutical and veterinary products, feed, feed supplements, personal care products and households products. Accordingly, the invention also relates to products comprising microcapsules of the invention.

The process of the invention may be carried out in accordance with the following general recipe or as shown in the examples:
The water soluble ingredients, including the matrix materials, are added to hot water and dissolved under continuous agitation. The oil soluble ingredients, if any, are mixed and then added to the water phase together with the active substance, and the mixture is treated to prepare a solution or dispersion. The solution or dispersion is diluted, if necessary, to an appropriate viscosity before the solution or dispersion is finely divided while simultaneously injecting a powdering agent and dried by a conventional method.
The invention will now be described in further detail with reference to the following examples.

### EXAMPLES

### Preparation of microcapsules

### Comparative Example 1

650 ml distilled water is heated to 65°C, where after 431 g sucrose and 407 g OSA-starch (Capsul® National Starch) is added under continuous agitation. When the sucrose and Capsul® has dissolved, 1220 g vitamin E acetate oil is added under vigorous stirring. The mixture is homogenised/emulsified for about 35 minutes at 5000 rpm. After emulsification 261 g of a 4% alginate solution in distilled water is added to the emulsion. The emulsion is diluted with distilled water to an appropriate sprayable viscosity.

Subsequently the emulsion is atomised in a spray drying tower, into which corn starch is simultaneously injected, and dried.

### Example 1: According to invention

650 ml distilled water is heated to 65°C, where after 431 g sucrose and 407 g OSA-starch (Capsul® National Starch) is added under continuous agitation. When the sucrose and Capsul® has dissolved, 1220 g vitamin E acetate oil is added under vigorous stirring. The mixture is homogenised/emulsified for about 35 minutes at 5000 rpm. After emulsification 261 g of a 4% alginate solution in distilled water is added to the emulsion. The emulsion is diluted with distilled water to an appropriate sprayable viscosity.

Subsequently the emulsion is atomised in a spray drying tower, into which a mixture of 90% corn starch and 10% Calcium Lactate is simultaneously injected, and dried. Hereby the alginate and the calcium ions form a complex.

### Comparative Example 2

2438 grams of fish gelatine (200 bloom, Rousselot 200 FG 30 from SKW Biosystems, France) and 2976 grams of sucrose are dissolved in 6.0 I demineralized water at 65°C in an emulsion tank and stored overnight to remove trapped air bubbles. A mixture of 2000 grams of Vitamin A Acetate 2.8 million IU/g and 140 g bytulated hydroxytoluene (BHT) is heated to 65°C in a beaker. The oily mixture is added to the aqueous solution of gelatine and sucrose under slow agitation, which is then vigorously agitated for 40 minutes at 65°C. Then the final emulsion is diluted with 1.5 I demineralised water to a viscosity of 164 cP. The mean oil droplet size is measured to be 0.29 µm. Subsequently the emulsion is atomised in a spray drying tower, into which corn starch is simultaneously injected, and dried.

### Example 2: According to invention

2438 grams of fish gelatine (200 bloom, Rousselot 200 FG 30 from SKW Biosystems, France) and 2976 grams of sucrose are dissolved in 6.0 I demineralised water at 65°C in an emulsion tank and stored overnight to remove trapped air bubbles. A mixture of 2000 grams of Vitamin A Acetate 2.8 million IU/g and 140 g bytulated hydroxytoluene (BHT) is heated to 65°C in a beaker. The oily mixture is added to the aqueous solution of gelatine and sucrose under slow agitation, which is then vigorously agitated for 40 minutes at 65°C. Then the final emulsion is diluted with 1.5 I demineralised water to a viscosity of 164 cP. The mean oil droplet size is measured to be 0.29 µm.

Subsequently the emulsion is atomised in a spray drying tower, into which a mixture of 90% corn starch and 10% glucose (reducing sugar) is simultaneously injected, and dried. Hereby the glucose reacts with the gelatine in a Maillard reaction.

### Example 3: According to invention

2438 grams of fish gelatine (200 bloom, Rousselot 200 FG 30 from SKW Biosystems, France) and 2976 grams of sucrose is dissolved in 6.0 I water at 65°C in an emulsion tank and stored overnight to remove trapped air bubbles. A mixture of 2000 grams of Vitamin A Acetate 2.8 million IU/g and 140 g bytulated hydroxytoluene (BHT) is heated to 65°C in a beaker. The oily mixture is added to the aqueous solution of gelatine and sucrose under slow agitation, which is then vigorously agitated for 40 minutes at 65°C. Then the final emulsion is diluted with 1.5 I water to a viscosity of 164 cP. The mean oil droplet size is measured to be 0.29 µm.

Subsequently the emulsion is atomised in a spray drying tower, into which corn starch mixed with a fine powder of transglutaminase (approx. 10U/g starch) is simultaneously injected, and dried. The drying must be sufficiently slow to let the cross-linking take place. Hereby the transglutaminase induces a cross-binding of the gelatine.

### Example 4: According to invention

2438 grams of fish gelatine (200 bloom, Rousselot 200 FG 30 from SKW Biosystems, France) and 2976 grams of sucrose is dissolved in 6.0 I water at 65°C in an emulsion tank and stored overnight to remove trapped air bubbles. A mixture of 2000 grams of Vitamin A Acetate 2.8 million IU/g and 140 g bytulated hydroxytoluene (BHT) is heated to 65°C in a beaker. The oily mixture is added to the aqueous solution of gelatine and sucrose under slow agitation, which is then vigorously agitated for 40 minutes at 65°C. Then the final emulsion is diluted with 1.5 I water to a viscosity of 164 cP. The mean oil droplet size is measured to be 0.29 µm.

Subsequently the emulsion is atomised in a spray drying tower, into which dialdehyde starch is simultaneously injected, and dried. The drying must be sufficiently slow to let the cross linking of the dialdehydes starch and gelatine takes place.

## Claims

1. Microcapsule comprising one or more active substances embedded in a matrix material and comprising a powdering agent, said microcapsule further comprising a component which is the product of the reaction between at least one component in said matrix material and at least one component in said powdering agent, and which microcapsule is obtainable by a process which comprises the steps of
- providing a solution or dispersion of said matrix material,
- adding to said solution or dispersion said at least one active substance,
- treating the mixture thus obtained to prepare a solution or dispersion of said at least one active substance and said matrix material,
- finely dividing and drying the solution or dispersion thus obtained to prepare a mass of particles each containing the active substance embedded in the matrix material,
- adding simultaneously with said finely dividing and drying step said powdering agent,
**characterised in that** said powdering agent comprises said at least one component, which is able to react with said at least one component in said matrix component.

2. Microcapsule according to claim 1, **characterised in that** said at least one matrix component and said at least one component in the powdering agent, which are able to react to provide the further component in the microcapsule, are selected from proteins, enzymes, mono-, di- and polysaccharides, anions, cations, acids, bases, cross-linking agents, such as dialdehydes and enzymes; polycations, polyanions, electrolytes, and water.

3. Microcapsule according to any of the claims 1 to 2, **characterised in that** one component is a protein, and another component is a sugar.

4. Microcapsule according to any of the claims 1 to 2, **characterised in that** one component is a protein, and another component is a dialdehyde.

5. Microcapsule according to any of the claims 1 to 2, **characterised in that** one component is a protein, and another component is an enzyme.

6. Microcapsule according to any of the claims 1 to 2, **characterised in that** one component is a polysaccharide, and another component is a cation.

7. Microcapsule according to any of the claims 1 to 2, **characterised in that** one component is a protein, and another component is water.

8. Microcapsule according to any of the claims 1 to 2, **characterised in that** one component is a protein, and another component is an acid.

9. Process of preparing microcapsules according to any of the claim 1 to 8, which process comprises the steps of
- providing a solution or dispersion of said matrix material;
- adding to said solution or dispersion said at least one active substance,
- treating the mixture thus obtained to prepare a solution or dispersion of said at least one active substance and said matrix material,
- finely dividing and drying the solution or dispersion thus obtained to prepare a mass of particles each containing the active substance embedded in said matrix,
- adding simultaneously with said finely dividing and drying step said powdering agent,
**characterised in that** said powdering agent comprises said at least one component, which is able to react with at least one component in said matrix component.

10. A product comprising microcapsules according to any of the claims 1 to 8.

11. A product comprising microcapsules obtainable according to claim 10.

12. A product according to any of the claims 10 and 11 **characterised in that** it is a food, a food supplement, a beverage, a pharmaceutical or veterinary product, a feed or feed supplement, a personal care product or a household product.
